(19) Europäisches Patentamt
European Patent Office
Office européen des brevets

(11) Publication number: **0 205 334**
**A2**

# (12) EUROPEAN PATENT APPLICATION

(21) Application number: 86304397.2

(22) Date of filing: 09.06.86

(51) Int. Cl.[4]: C 07 D 223/16
A 61 K 31/55

(30) Priority: 12.06.85 US 743841
22.11.85 US 800936

(43) Date of publication of application:
17.12.86 Bulletin 86/51

(84) Designated Contracting States:
AT BE CH DE FR GB IT LI LU NL SE

(71) Applicant: E.R. Squibb & Sons, Inc.
Lawrenceville-Princeton Road
Princeton, N.J. 08540(US)

(72) Inventor: Floyd, David M.
R.R. No. 1 Box 404M
Pennington New Jersey(US)

(74) Representative: Thomas, Roger Tamlyn et al,
D. Young & Co. 10 Staple Inn
London WC1V 7RD(GB)

(54) **Benzazepine derivatives.**

(57) Vasodilating activity is exhibited by new compounds having the formula

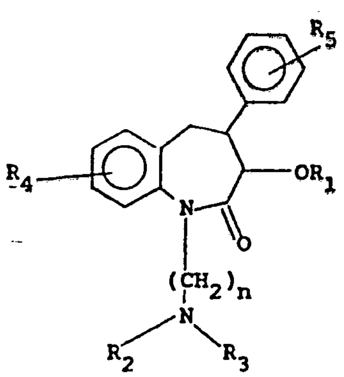

and pharmaceutically acceptable salts thereof, wherein $R_1$, $R_2$, $R_3$, $R_4$, $R_5$ and n are as defined herein.

EP 0 205 334 A2

## BENZAZEPINE DERIVATIVES

This invention relates to new compounds having the formula

I

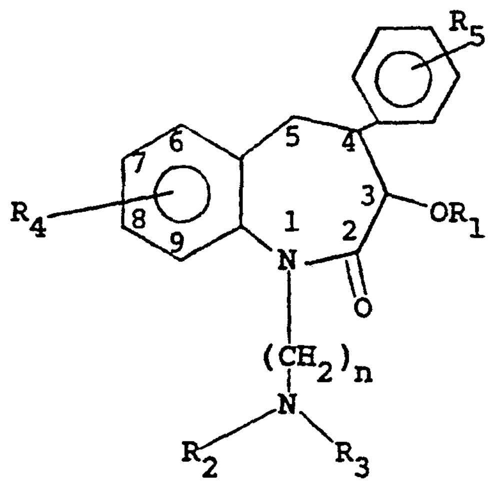

and the pharmaceutically acceptable salts thereof. These new compounds have useful vasodilating activity. In formula I, and throughout the specification, the symbols are as defined below.

$R_1$ is hydrogen, alkyl, alkanoyl, alkenyl, arylcarbonyl, heteroarylcarbonyl or $-\overset{O}{\overset{\|}{C}}-NX_1X_2$;

$R_2$ and $R_3$ are each independently alkyl or cycloalkyl or $R_2$ and $R_3$ together with the nitrogen atom to which they are attached are pyrrolidinyl, piperidinyl, or morpholinyl;

$R_4$ and $R_5$ are each independently hydrogen, halogen, alkyl, alkoxy, aryloxy, arylalkoxy,

arylalkyl, hydroxy, alkanoyloxy, $-O-\overset{O}{\overset{\|}{C}}-NX_1X_2$, difluoromethoxy, trifluoromethyl, $-NX_3X_4$, $-S(O)_m$alkyl, or $-S(O)_m$aryl;

n is 2 or 3;

m is 0, 1 or 2;

$X_1$ and $X_2$ are each independently hydrogen, alkyl, aryl or heteroaryl, or $X_1$ and $X_2$ together with the nitrogen atom to which they are attached are a nitrogen containing heteroaryl;

$X_3$ and $X_4$ are each independently alkyl, alkanoyl, arylcarbonyl, heteroarylcarbonyl, or carbamoyl ($H_2N-\overset{O}{\overset{\|}{C}}-$);

with the proviso that if $R_4$ is a 7-alkyl group, it must have a tertiary carbon atom bonded to the ring.

Listed below are definitions of various terms used to describe the benzazepines of this invention. These definitions apply to the terms as they are used throughout the specification (unless they are otherwise limited in specific instances) either individually or as part of a larger group.

The terms "alkyl" and "alkoxy" refer to both straight and branched chain groups. Those groups having 1 to 10 carbon atoms are preferred.

The term "alkenyl" refers to both straight and branched chain groups. Those groups having 2 to 10 carbon atoms are preferred.

The term "aryl" refers to phenyl and substituted phenyl. Exemplary substituted phenyl groups are phenyl groups substituted with 1, 2 or 3 amino ($-NH_2$), alkylamino, dialkylamino, nitro, halogen, hydroxyl, trifluoromethyl, alkyl (of 1 to 4 carbon atoms), alkoxy (of 1 to 4 carbon atoms),

alkanoyloxy, carbamoyl, or carboxyl groups.

The term "alkanoyl" refers to groups having the formula $\text{alkyl-}\overset{O}{\overset{\|}{C}}\text{-}$. Those alkanoyl groups having 2 to 11 carbon atoms are preferred.

The term "heteroaryl" refers to an aromatic heterocyclic group having at least one heteroatom in the ring. Preferred groups are pyridinyl, pyrrolyl, imidazolyl, furyl, thienyl, or thiazolyl.

The term "nitrogen containing heteroaryl" refers to an aromatic heterocyclic group having at least one nitrogen atom in the ring. Preferred groups are pyridinyl, pyrrolyl, imidazolyl and thiazolyl.

The term "cycloalkyl" refers to groups having 3, 4, 5, 6 or 7 carbon atoms.

The term "halogen" refers to fluorine, chlorine, bromine and iodine.

The compounds of formula I form acid-addition salts with inorganic and organic acids. These acid-addition salts frequently provide useful means for isolating the products from reaction mixtures by forming the salt in a medium in which it is insoluble. The free base may then be obtained by neutralization, e.g., with a base such as sodium hydroxide. Any other salt may then be formed from the free base and the appropriate inorganic or organic acid. Illustrative are the hydrohalides, especially the hydrochloride and hydrobromide, sulfate, nitrate, phosphate, borate, acetate, tartrate, maleate, citrate, succinate, benzoate, ascorbate, salicylate, methanesulfonate, benzenesulfonate, toluenesulfonate and the like.

The carbon atoms in the 3 and 4-positions of the benzazepine nucleus of the compound of formula I are asymmetric carbons. The compounds of formula I,

therefore, exist in enantiomeric and diastereomeric forms and as racemic mixtures thereof. All are within the scope of this invention. It is believed that those compounds of formula I which have the d-cis configuration are the most potent and are therefore preferred.

The compounds of formula I and the pharmaceutically acceptable salts thereof are useful as cardiovascular agents. These compounds act as vasodilators and are especially useful as anti-hypertensive agents. By the administration of a composition containing one (or a combination) of the compounds of this invention the blood pressure of a hypertensive mammalian (e.g., human) host is reduced. Daily doses of about 0.1 to 100 mg. per kilogram of body weight per day, preferably about 1 to about 50 mg. per kilogram per day, are appropriate to reduce blood pressure, and can be administered in single or divided doses. The substance is preferably administered orally, but parenteral routes such as the subcutaneous, intramuscular, or intravenous routes can also be employed.

As a result of the vasodilating activity of the compounds of formula I, it is believed that such compounds in addition to being anti-hypertensives may also be useful as anti-arrhythmic agents, as anti-anginal agents, as anti-fibrillatory agents, as anti-asthmatic agents, and in limiting myocardial infarction.

The compounds of this invention can also be formulated in combination with a diuretic, or a beta-adrenergic agent, or angiotensin converting enzyme inhibitor. Suitable diuretics include the

thiazide diuretics such as hydrochlorothiazide and bendroflumethiazide, suitable beta-adrenergic agents include nadolol, and suitable angiotensin converting enzyme inhibibors include captopril.

The compounds of formula I can be prepared by first reacting a 2-nitrotoluene having the formula

II

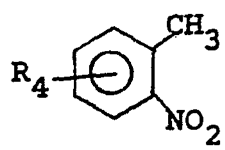

with a benzylidine malonate having the formula

III

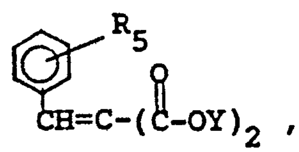

wherein Y is alkyl. The reaction can be run in a polar nonprotic solvent (e.g., dimethylformamide), in the presence of a strong base such as sodium hydride, and yields a product having the formula

IV

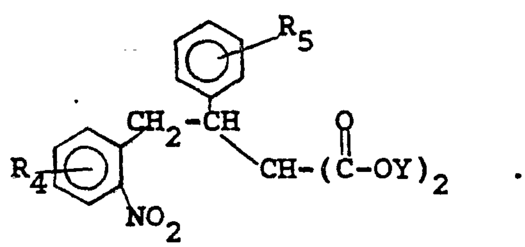

Reduction of a compound of formula IV yields the corresponding compound having the formula

V

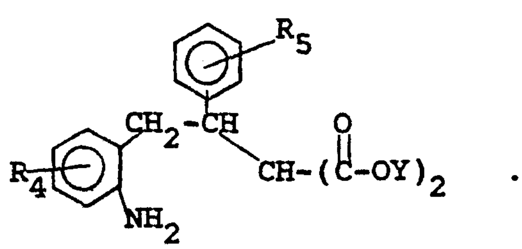

The reduction can be accomplished by catalytic hydrogenation (using, for example, palladium on charcoal as a catalyst) or using a chemical

reducing agent (e.g., ferrous sulfate or metallic tin).

Treatment of an amine of formula V with an alkali metal alkoxide (e.g., sodium methoxide) and an alcohol (e.g., methanol) yields the corresponding benzazepine having the formula

VI

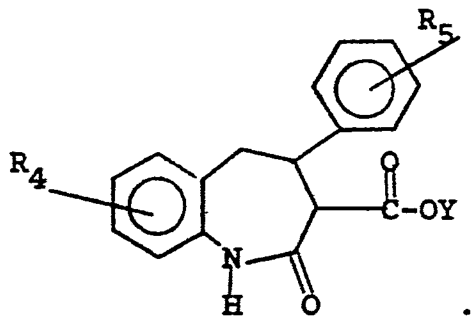

Reaction of a compound of formula VI with a strong base (e.g., lithium diisopropylamide or potassium hexamethyldisilazide) in an etheral solvent, such as tetrahydrofuran, at low temperature in the presence of anhydrous oxygen gas yields the corresponding compound having the formula

VII

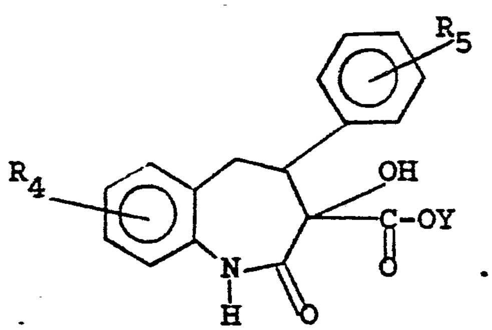

Alternatively, a compound of formula VII can be prepared by first cooling a compound of formula VI to a greatly reduced temperature (e.g., about -78°C) in a solvent such as tetrahydrofuran and treating it with a strong base (e.g., lithium diisopropylamide or potassium hexamethyldisilazide). Treatment of the compound with anhydrous oxygen gas in the presence of triethyl phosphite yields the desired compound of formula VII.

Decarboxylation of a compound of formula VII can be accomplished by treating the compound with excess lithium iodide in hot pyridine to obtain a mixture of isomers having the formulas

VIIIa

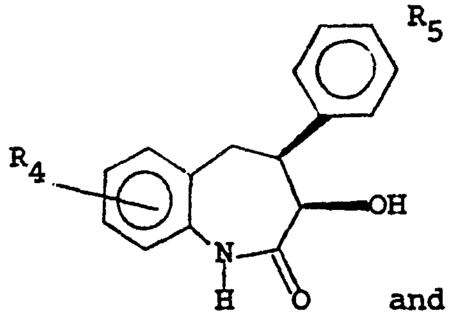

and

cis isomer

VIIIb

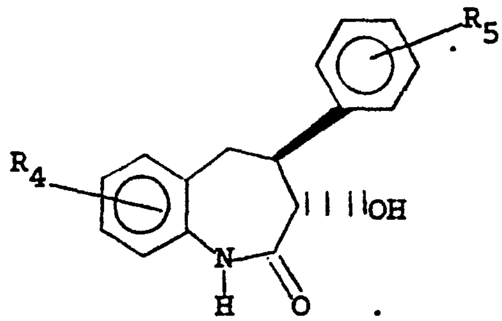

.

trans isomer

The preferred cis isomer is generally the predominant isomer formed during the above reaction. The isomers can be separated using art recognized techniques such as crystallization or chromatography. Alternatively, the reactions described hereinafter can be run using the diastereomeric mixture (mixture of compounds of formulas VIIIa and VIIIb). The isomeric mixture can be separated into its component isomers at any point during the reaction sequence.

Treatment of a compound of formula VIIIa or VIIIb with an alkali metal hydride (e.g., sodium hydride) in an inert solvent such as dimethylformamide or dimethylsulfoxide, followed by reaction with a compound having the formula

IX halogen-$(CH_2)_n$-$NR_2R_3$,

yields the corresponding compound having the formula

X

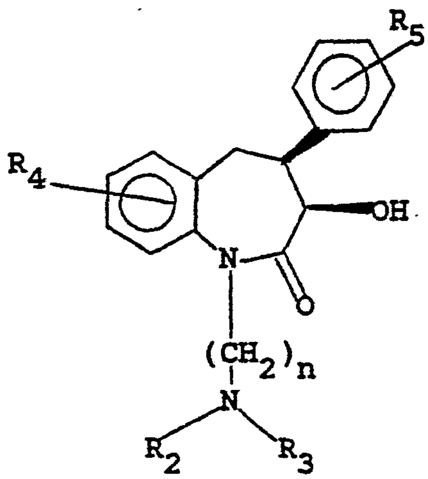

or corresponding trans isomer; i.e., a product of formula I wherein $R_1$ is hydrogen.

The compounds of formula X (or corresponding trans isomer) can be acylated or alkylated using conventional techniques to obtain those products of formula I wherein $R_1$ is other than hydrogen. For example, a compound of formula X (or corresponding trans isomer) can be reacted with a halide of the formula

XI $R_1$-halogen

in the presence of a base. Alternatively, the acylation can be accomplished using an acid anhydride.

Preferred members of each of the substituent groups of the benzazepines of formula I are as follows: $R_1$ is acetyl; n is 2; $R_2$ and $R_3$ are each methyl; $R_4$ is hydrogen or halogen (especially 7-chloro); and $R_5$ is 4-methoxy.

The following examples are specific embodiments of this invention.

## Example 1

**(cis)-3-(Acetyloxy)-7-chloro-1-[2-(dimethylamino)-ethyl]-1,3,4,5-tetrahydro-4-(4-methoxyphenyl)-2H-1-benzazepin-2-one, monohydrochloride**

### METHOD I

A) [2-(5-Chloro-2-nitrophenyl)-1-(4-methoxyphenyl)ethyl]propanedioic acid, dimethyl ester

To a stirred mixture of dimethyl p-methoxybenzylidene malonate (40 g, 0.16 mole) and 60% dispersion of sodium hydride (9.6 g, 0.24 mole) in 350 ml of dry dimethylformamide, was added dropwise over 2 hours a solution of 5-chloro-2-nitrotoluene (30 g, 0.176 mole) in 30 ml of dimethylformamide. The reaction was stirred at room temperature for 6 hours, then quenched with glacial acetic acid (15.4 ml, 0.26 mole). The solvent was removed in vacuo and the residue was triturated with water. The yellow solids were filtered and triturated with methanol to yield 50.3 g of a white solid, melting point 128.5-130.5°C.

B) [2-(2-Amino-5-chlorophenyl)-1-(4-methoxyphenyl)ethyl]propanedioic acid, dimethyl ester

To a refluxing mixture of [2-(5-chloro-2-nitrophenyl)-1-(4-methoxyphenyl)ethyl]propanedioic acid, dimethyl ester (40 g, 95.0 mmole) and hydrated ferrous sulfate (184.5 g, 0.663 mole) in a (1:10 solution of methanol:water (1.2 L) was added concentrated ammonium hydroxide (142.5 ml) over a 30 minute period. The reaction was stirred at reflux for 20 minutes then cooled to room temperature. Ethyl acetate and Celite were added and the mixture was filtered through Celite. The filtrate was partitioned between ethyl acetate and

water. The organic phase was dried over magnesium sulfate and concentrated in vacuo. The product was recrystallized from isopropyl alcohol to yield 28.22 g of the title compound, melting point 114-116°C.

C) 7-Chloro-1,3,4,5-tetrahydro-3-(methoxycarbonyl)-4-(4-methoxyphenyl)-2H-1-benzazepin-2-one

To a solution of [2-(2-amino-5-chlorophenyl)-1-(4-methoxyphenyl)ethyl]propanedioic acid, dimethyl ester (23.2 g, 59.2 mmole) in methanol (200 ml) was added a 25% solution of sodium methoxide in methanol (16 ml, 69.97 mmole). The solution was refluxed for 3 hours under argon. The reaction was cooled to room temperature and treated with 200 ml 1N hydrochloric acid. A white precipitate was filtered and washed with water, methanol, and dried in vacuo to yield 19.5 g of the title compound, melting point 189-190.5°C.

D) 7-Chloro-1,3,4,5-tetrahydro-3-hydroxy-3-(methoxycarbonyl)-4-(4-methoxyphenyl)-2H-1-benzazepin-2-one

Oxygen was bubbled through a solution of 7-chloro-1,3,4,5-tetrahydro-3-(methoxycarbonyl)-4-(4-methoxyphenyl)-2H-1-benzazepin-2-one (7.0 g, 19.46 mmole) in 100 ml of dry tetrahydrofuran at 0°C while a 0.67 M solution of potassium hexamethyldisilazide in toluene (87.64 ml, 58.76 mmole) was added dropwise over 15 minutes. The reaction was continued for 1 hour with the continued bubbling of oxygen at 0°C. The reaction was quenched with 75 ml of a 5% solution of potassium bisulfate, followed by the addition of solid sodium sulfite and 10 ml of methanol. The reaction was

stirred for 30 minutes and extracted twice with ethyl acetate. The combined organic layers were washed with 1N hydrochloric acid, saturated sodium bicarbonate, and dried over magnesium sulfate. The solvent was removed *in vacuo* and the residue was recrystallized from isopropyl alcohol to yield 4.5 g of the title compound.

E) (*cis*)-7-Chloro-1,3,4,5-tetrahydro-3-hydroxy-4-(4-methoxyphenyl)-2H-1-benzazepin-2-one

A solution of 7-chloro-1,3,4,5-tetrahydro-3-hydroxy-3-(methoxycarbonyl)-4-(4-methoxyphenyl)-2H-1-benzazepin-2-one (0.5 g, 1.33 mmole) and lithium iodide (0.178 g, 1.33 mmole) in dry pyridine (20 ml) was refluxed under argon until complete conversion to product by TLC. The reaction was cooled to room temperature and concentrated *in vacuo*. The residue was dissolved in chloroform and extracted twice with 1N hydrochloric acid, once with saturated sodium chloride, and dried over magnesium sulfate. The solvent was removed *in vacuo* and the residue was triturated with ether to yield 0.22 g of the cis product.

F) (*cis*)-7-Chloro-3-hydroxy-1-[2-(dimethylamino)ethyl]-1,3,4,5-tetrahydro-4-(4-methoxyphenyl)-2H-1-benzazepin-2-one, monohydrochloride

To a solution of (*cis*)-7-chloro-1,3,4,5-tetrahydro-3-hydroxy-4-(4-methoxyphenyl)-2H-1-benzazepin-2-one (0.83 g, 2.612 mmole) in dry dimethylformamide (26.12 ml) was added a 60% dispersion of sodium hydride (0.11 g, 2.74 mmole). The reaction was stirred at room temperature under argon for 1 hour and a 1.7N

solution of dimethylaminoethyl chloride in toluene (2.30 ml, 3.92 mmole) was added and stirred at 75°C for 2 hours. The solvent was removed *in vacuo* and the residue was partitioned between ethyl acetate and 1*N* hydrochloric acid. The organic phase was washed with 1*N* hydrochloric acid, and dried over magnesium sulfate. The aqueous phase was treated with 6*N* sodium hydroxide to adjust to pH 11. The product was extracted three times with ethyl acetate and dried over magnesium sulfate. The solvent was removed *in vacuo*. The residue was dissolved in 2 ml of ethyl acetate and 5 ml of ether and treated with 1.2 equivalents of 1.39*N* hydrochloric acid in ether at 0°C. The solids were filtered and washed with ether to yield 0.65 g of the title compound.

G) (*cis*)-3-(Acetyloxy)-7-chloro-1-[2-(dimethylamino)ethyl]-1,3,4,5-tetrahydro-4-(4-methoxyphenyl)-2H-1-benzazepin-2-one, monohydrochloride

A solution of (*cis*)-7-chloro-3-hydroxy-1-[2-(dimethylamino)ethyl]-1,3,4,5-tetrahydro-4-(4-methoxyphenyl)-2H-1-benzazepin-2-one, monohydrochloride (0.62 g, 1.5 mmole) in dry acetic anhydride (25 ml) was heated at 110°C under argon, until judged complete by TLC. The solvent was removed *in vacuo* and the residue was dissolved in ethyl acetate and 2 ml of saturated hydrochloric acid in ether was added. A white precipitate was filtered and dried (yield 0.5 g). The reaction was repeated to afford an additional 0.27 g of product. The batches were combined, melting point 207.5°C-209°C.

Analysis Calc'd. for $C_{23}H_{28}N_2Cl_2O_4 \cdot 0.7$ mole $H_2O$:
C, 57.55; H, 6.17; N, 5.84; Cl, 14.77
Found: C, 57.54; H, 5.86; N, 5.63; Cl, 14.92

METHOD II

Method II is identical with the above-described Method I except for the following parts D and E:

D) 7-Chloro-1,3,4,5-tetrahydro-3-hydroxy-3-(methoxycarbonyl)-4-(4-methoxyphenyl)-2H-1-benzazepin-2-one

A solution of 7-chloro-1,3,4,5-tetrahydro-3-(methoxycarbonyl)-4-(4-methoxyphenyl)-2H-1-benzazepin-2-one (15 g, 41.7 mmole) in 780 ml of tetrahydrofuran was cooled to -78°C and 147 ml (167 mmole in tetrahydrofuran) of potassium hexamethyldisilazide solution was added. After stirring for 1 hour, 28.7 ml of triethyl phosphite (166.7 mmole) was added and anhydrous oxygen gas was rapidly bubbled through the resulting solution. The reaction temperature was then raised to 0°C and allowed to stir for an additional hour. Oxygenation was then discontinued and the reaction was quenched by the addition of 500 ml of acetic acid. The reaction mixture was then concentrated and the residue dissolved in ethyl acetate. The organic solution was washed successively with 1N hydrochloric acid, saturated sodium bicarbonate, and brine and then dried over anhydrous sodium sulfate. Concentration of the dried organic solution afforded a solid which, upon trituration in hexane, gave 14.8 g of the title compound.

E) (*cis*)-7-Chloro-1,3,4,5-tetrahydro-3-hydroxy-4-(4-methoxyphenyl)-2H-1-benzazepin-2-one

A solution of 7-chloro-1,3,4,5-tetrahydro-3-hydroxy-3-(methoxcarbonyl)-4-(4-methoxyphenyl)-2*H*-1-benzazepin-2-one (16.4 g, 43.7 mmole) and lithium iodide (23.7 g, 174.6 mmole) in 290 ml of pyridine containing 1% water was refluxed under argon until complete conversion to product by TLC (ca. 1.5 hours). The reaction was cooled to room temperature and concentrated *in vacuo*. The residue was dissolved in ethyl acetate and extracted with 1*N* hydrochloric acid, saturated sodium bicarbonate and saturated sodium chloride. The solution was dried over magnesium sulfate and concentrated. The residue was recrystallized from ethyl acetate to afford 6.8 g of pure *cis* product.

## Example 2

(*cis*)-3-(Acetyloxy)-1-[2-(dimethylamino)ethyl]-1,3,4,5-tetrahydro-4-(4-methoxyphenyl)-2*H*-1-benzazepin-2-one, monohydrochloride

A) [1-(4-Methoxyphenyl)-2-(2-nitrophenyl)ethyl]-propanedioic acid, dimethyl ester

Dimethyl p-methoxybenzylidene malonate (90 g, 360 mmol) was dissolved in 700 ml of dry dimethylformamide under argon and 21.6 g (540 mmol) of sodium hydride as a 60% dispersion in oil was added. The suspension was then treated at room temperature with 33.9 ml (400 mmol) of 2-nitrotoluene and a catalytic amount of t-butanol was added. After stirring for 16 hours, the reaction was quenched by the addition of 3L of 1*N* hydrochloric acid and extracted four times with ether. The combined organic phases were dried

over sodium sulfate and evaporated to yield 171 g of dark oil. Chromatography on 1.5 kg of 60-200 silica with 4:1 hexane-ethyl acetate gave 22 g of high purity product and an additional 22 g of somewhat impure material as judged by tlc analysis.

B) [2-(2-Aminophenyl)-1-(4-methoxyphenyl)ethyl]-propanedioic acid, dimethyl ester

Hydrogenation of 22 g of [1-(4-methoxyphenyl)-2-(2-nitrophenyl)ethyl]propanedioic acid, dimethyl ester in 450 ml of 5:1 methanol-acetic acid at atmospheric pressure for 16 hours followed by recrystallization of the crude product from isopropanol gave 13 g of the desired product. Chromatography of the concentrated mother liquor on LPS-1 silica with 7:3 hexane-ethyl acetate gave an additional 2.24 g of product.

C) 1,3,4,5-tetrahydro-3-(Methoxycarbonyl)-4-(4-methoxyphenyl)-2H-1-benzazepin-2-one

A solution containing 11.97 (33.5 mmol) of [2-(2-aminophenyl)-1-(4-methoxyphenyl)ethyl]-propanedioic acid, dimethyl ester and 9.2 ml of 25% sodium methoxide (40.2 mmol) in 80 ml of methanol was refluxed for 1.5 hours and cooled to room temperature. Excess 1M hydrochloric acid solution was then added and the resulting precipitated product was removed by filtration to give 9.18 g of pure material, melting point 217-219°C.

D) 1,3,4,5-tetrahydro-3-Hydroxy-3-(methoxycarbonyl)-4-(4-methoxyphenyl)-2H-1-benzazepin-2-one

Dry oxygen gas was bubbled through a cooled (ice bath) solution of 1,3,4,5-tetrahydro-3-(methoxycarbonyl)-4-(4-methoxyphenyl)-2H-1-benzazepin-2-one (13 g, 40.2 mmol) in 130 ml of dry tetrahydrofuran for 30 minutes. Potassium hexamethyldisilazide in toluene (180 ml, 0.67M, 120.5 mmol) was then added over a 7 minute period under a continuous stream of oxygen. After stirring for 2.5 hours, the oxygen flow was terminated and the reaction was quenched by the addition of 200 ml of 5% potassium bisulfate. Solid sodium sulfite (15 g) was then added followed by the addition of 100 ml of methanol and the mixture was stirred for an additional 30 minutes. The mixture was then extracted twice with ethyl acetate and the combined organic layers were washed three times with 1M hydrochloric acid, once with saturated bicarbonate solution followed by brine and then dried over sodium sulfate. Concentration afforded 15 g of crude product which was triturated with ether-hexane to yield 9.3 g of high purity product.

E) (cis)-1,3,4,5-tetrahydro-3-Hydroxy-4-(4-methoxyphenyl)-2H-1-benzazepin-2-one

A solution containing 5.26 g (14.6 mmol) of 1,3,4,5-tetrahydro-3-hydroxy-3-(methoxycarbonyl)-4-(4-methoxyphenyl)-2H-1-benzazepin-2-one and 19.6 g (147 mmol) of anhydrous lithium iodide in 150 ml of dry pyridine was refluxed for one hour. After concentration, the crude product was dissolved in ethyl acetate, washed with 6M hydrochloric acid

followed by saturated bicarbonate and dried over sodium sulfate. Concentration followed by trituration with ether gave 2.81 g of essentially pure cis isomer, melting point 173.5-175.5°C (dec).

F) (cis)-3-Hydroxy-1-[2-(dimethylamino)ethyl]-1,3,4,5-tetrahydro-4-(4-methoxyphenyl)-2H-1-benzazepin-2-one, monohydrochloride

A solution containing 1.0 g of (cis)-1,3,4,5-tetrahydro-3-hydroxy-4-(4-methoxyphenyl)-2H-1-benzazepin-2-one (3.53 mmol) in 35 ml of dry dimethylformamide was treated with 0.15 g (3.71 mmol) of sodium hydride as a 60% dispersion in oil and stirred for one hour. A solution of N,N-dimethyl-2-chloroethyl amine in toluene (3.1 ml of 1.7M solution, 5.3 mmol) was then added and the mixture was heated at 75°C for 1.5 hours. The reaction was then concentrated on a vacuum pump and the residue was partitioned between ethyl acetate and 1M hydrochloric acid. The organic phase was washed twice more with the aqueous hydrochloric acid and the combined aqueous phases were adjusted to pH 11 and extracted three times with ethyl acetate. The combined ethyl acetate layers were dried over sodium sulfate and concentrated to yield 1.17 g of the desired material. The crude product was dissolved in a minimal amount of chloroform and acidified at 0°C with saturated ether/hydrogen chloride solution resulting in the formation of 1.27 g of an off-white powder.

G) (cis)-3-(Acetyloxy)-1-[2-(dimethylamino)ethyl]-1,3,4,5-tetrahydro-4-(4-methoxyphenyl)-2*H*-1-benzazepin-2-one, monohydrochloride

(*cis*)-3-Hydroxy-1-[2-(dimethylamino)ethyl]-1,3,4,5-tetrahydro-4-(4-methoxyphenyl)-2*H*-1-benzazepin-2-one, monohydrochloride (1.27 g, 2.83 mmol) was slurried in 28 ml of acetic anhydride and heated at 100°C under argon for 3 hours. The resulting solution was then concentrated and the residue dissolved in ethyl acetate. After crystallization commenced, hydrogen chloride saturated ether was added and the mixture was filtered to afford 1.25 g of the desired material as an off-white powder, melting point 215-216°C.

Analysis Calc'd. for $C_{23}H_{29}ClN_2O_4 \cdot 0.4$ mole $H_2O$:
C, 62.76; H, 6.82; N, 6.36; Cl, 8.05
Found: C, 62.76; H, 6.65; N, 6.40; Cl, 7.97

## Example 3

(*trans*)-3-(Acetyloxy)-1-[2-(dimethylamino)ethyl]-1,3,4,5-tetrahydro-4-(4-methoxyphenyl)-2H-1-benzazepin-2-one, monohydrochloride

A) (*trans*)-3-Hydroxy-1,3,4,5-tetrahydro-4-(4-methoxyphenyl)-2H-1-benzazepin-2-one

Flash chromatography (LPS-1 silica gel, 70:30 hexanes-ethyl acetate) of the mother liquor resulting from the trituration from ether of the crude (*cis*)-3-hydroxy-1,3,4,5-tetrahydro-4-(4-methoxyphenyl)-2H-1-benzazepin-2-one (see example 2E) afforded the title *trans* isomer, melting point 168-170°C.

B) (trans)-3-Hydroxy-1-[2-(dimethylamino)ethyl]-1,3,4,5-tetrahydro-4-(4-methoxyphenyl)-2H-1-benzazepin-2-one

Following the procedure of example 2F, but substituting 0.57 g (2.01 mmol) of (trans)-3-hydroxy-1,3,4,5-tetrahydro-4-(4-methoxyphenyl)-2H-1-benzazepin-2-one for the corresponding cis isomer, yielded 0.66 g of the title compound as an oil.

C) (trans)-3-(Acetyloxy)-1-[2-(dimethylamino)-ethyl]-1,3,4,5-tetrahydro-4-(4-methoxyphenyl)-2H-1-benzazepin-2-one, monohydrochloride

Following the procedure of example 2G, but substituting 0.654 g (1.83 mmol) of (trans)-3-hydroxy-1-[2-(dimethylamino)ethyl]-1,3,4,5-tetrahydro-4-(4-methoxyphenyl)-2H-1-benzazepin-2-one for the monohydrochloride salt of the corresponding cis isomer yielded 0.5 g of the title compound as a white crystalline solid after trituration from ethyl acetate and recrystallization from methanol/isopropanol. The product melted at 254-256.5°C.

Analysis Calc'd for $C_{23}H_{29}ClN_2O_4 \cdot 0.2$ mole of $H_2O$:
C, 63.31; H, 6.79; N, 6.42; Cl, 8.12
Found: C, 63.31; H, 6.90; N, 6.29; Cl, 8.13

Example 4-8

Following the procedure of Example 1, Method II, but substituting the compound listed in Column I for dimethyl p-methoxybenzylidene malonate, yielded the compound listed in Column II.

| Column I | Column II |
|---|---|
| 4. dimethyl p-(trifluoromethyl)-benzylidene malonate | (cis)-3-(Acetyloxy)-7-chloro-1-[2-(dimethylamino)-ethyl]-1,3,4,5-tetrahydro-4-[4-(trifluoromethyl)-phenyl]-2H-1-benzazepin-2-one, monohydrochloride, melting point 225.5-227°C, dec. |
| 5. dimethyl benzylidene malonate | (cis)-3-(Acetyloxy)-7-chloro-1-[2-(dimethylamino)ethyl]-1,3,4,5-tetrahydro-4-phenyl-2H-1-benzazepin-2-one, monohydrochloride, melting point 247-250°C, dec. |
| 6. dimethyl o-methoxybenzylidene malonate | (cis)-3-(Acetyloxy)-7-chloro-1-[2-(dimethylamino)ethyl]-1,3,4,5-tetrahydro-4-(2-methoxyphenyl)-2H-1-benzazepin-2-one, monohydrochloride, melting point 250-252°C, dec. |
| 7. dimethyl m-methoxybenzylidene malonate | (cis)-3-(Acetyloxy)-7-chloro-1-[2-(dimethylamino)ethyl]-1,3,4,5-tetrahydro-4-(3-methoxyphenyl)-2H-1-benzazepin-2-one, monohydrochloride, melting point 225-226°C. |

| | |
|---|---|
| 8. dimethyl p-chloro-benzylidene malonate | (cis)-3-(Acetyloxy)-7-chloro-4-(4-chlorophenyl)-1-[2-(dimethylamino)ethyl]-1,3,4,5-tetrahydro-2H-1-benzazepin-2-one, methanesulfonate (1:1) salt, melting point 250-250.5°C. |

Examples 9-13

Following the procedure of Example 1, Method II, but substituting the compound listed in Column I for 5-chloro-2-nitrotoluene, yielded the compound listed in Column II.

| Column I | Column II |
|---|---|
| 9. 6-methyl-2-nitro-toluene | (cis)-3-(Acetyloxy)-1-[2-(dimethylamino)ethyl]-1,3,4,5-tetrahydro-4-(4-methoxyphenyl)-6-methyl-2H-1-benzazepin-2-one, monohydrochloride, melting point 176-178°C (sintering at 172°C). |
| 10. 5-(trifluoro-methyl)-2-nitro-toluene | (cis)-3-(Acetyloxy)-1-[2-(dimethylamino)ethyl]-1,3,4,5-tetrahydro-4-(4-methoxyphenyl)-7-(tri-fluoromethyl)-2H-1-benza-zepin-2-one, monohydro-chloride, melting point 230-232°C, dec., sintering at 227°C. |

| | |
|---|---|
| 11. 6-chloro-2-nitrotoluene | (cis)-3-(Acetyloxy)-6-chloro-1-[2-(dimethylamino)-ethyl]-1,3,4,5-tetrahydro-4-(4-methoxyphenyl)-2H-1-benzazepin-2-one, monohydrochloride, melting point 151-153°C, sintering at 148°C. |
| 12. 4-chloro-2-nitrotoluene | (cis)-3-(Acetyloxy)-8-chloro-1-[2-(dimethylamino)ethyl]-1,3,4,5-tetrahydro-4-(4-methoxyphenyl)-2H-1-benzazepin-2-one, monohydrochloride, melting point 173-176°C, sintering at 164°C. |
| 13. 5-(phenylmethoxy)-2-nitrotoluene | (cis)-3-(Acetyloxy)-1-[2-(dimethylamino)ethyl]-1,3,4,5-tetrahydro-4-(4-methoxyphenyl)-7-(phenylmethoxy)-2H-1-benzazepin-2-one, monohydrochloride, melting point 117-120°C, sintering at 107°C. |
| 14. 6-(trifluoromethyl)-2-nitrotoluene | (cis)-3-(Acetyloxy)-1-[2-(dimethylamino)ethyl]-1,3,4,5-tetrahydro-4-(4-methoxyphenyl)-6-(trifluoromethyl)-2H-1-benzazepin-2-one,monohydrochloride, melting point 222 - 224°C, dec. |

Example 15

(cis)-3-(Acetyloxy)-1-[2-(dimethylamino)ethyl]-1,3,4,5-tetrahydro-7-hydroxy-4-(4-methoxyphenyl)-2H-1-benzazepin-2-one, monohydrochloride

A solution of 0.5 g (0.87 mmol) of (cis)-3-(acetyloxy)-1-[2-(dimethylamino)ethyl]-1,3,4,5-tetrahydro-4-(4-methoxyphenyl)-7-(phenylmethoxy)-2H-1-benzazepin-2-one, monohydrochloride, (see Example 13) in 30 ml of methanol was treated under argon with 150 mg of 10% palladium on charcoal and shaken on the Parr hydrogenator at 50 p.s.i. for 5 hours. The catalyst was filtered off under argon, washed with methanol and the combined filtrates evaporated. The residue (which began to solidify) was rubbed under ether and the evaporation repeated. The solid was dried in vacuo for several hours yielding 0.40 g of the title compound, melting point 229-231°C, dec., preceded by gradual sintering.

Analysis calc'd. for $C_{23}H_{28}N_2O_5 \cdot HCl \cdot 1.5MH_2O$:

C, 58.04; H, 6.58; N, 5.89; Cl, 7.45

Found: C, 58.29; H, 6.33; N, 5.70; Cl, 7.63

Example 16

(cis)-7-Chloro-3-[(2-chloro-4-nitrobenzoyl)oxy]-1-[2-(dimethylamino)ethyl]-1,3,4,5-tetrahydro-4-(4-methoxyphenyl)-2H-1-benzazepin-2-one, methanesulfonate (1:1) salt

A solution of 0.80 g (2.06 mmol) of (cis)-7-chloro-3-hydroxy-1-[2-(dimethylamino)ethyl]-1,3,4,5-tetrahydro-4-(4-methoxyphenyl)-2H-1-benzazepin-2-one (see Example 1F) was treated with 0.65 g (3.2 mmol) of 2-chloro-4-nitrobenzoic acid, 0.52 g (2.5 mmol) of dicyclohexylcarbodiimide and

0.04 g of 4-dimethylaminopyridine. A precipitate of dicyclohexylurea separated from solution. After standing overnight at room temperature, the reaction was filtered. The filtrate was extracted with 10 ml of 10% potassium carbonate, 5 ml of 5% potassium carbonate (twice), 5 ml of water and 5 ml of saturated sodium chloride solution. The organic phase was dried (magnesium sulfate), filtered and the solvent evaporated to give 1.3 g of a pale yellow solid. The mixture was purified by chromatography on 50 g of silica gel using 1:1 dichloromethane-ethyl acetate as the solvent to give 1.0 g of the cis ester containing a small amount of the trans ester. The mixture was suspended in 5 ml of ether, cooled and filtered to give 0.9 g of nearly colorless solid. The small quantity of the trans ester still present was removed by suspending and stirring in 3 ml of isopropanol for 1 hour at room temperature, filtering and washing with isopropanol and ether to give 0.76 g of the free base, melting point 164-166°C. The material was converted to the methanesulfonate salt by dissolving it in 10 ml of warm ethyl acetate and treating with a solution of 0.135 g of methanesulfonate in 5 ml of ethyl

acetate. The resulting solution was gradually diluted with ether to give a colorless crystalline product. After cooling overnight, the solid was filtered, washed with ether and dried; weight 0.84 g, melting point 215-217°C.

Analysis Calc'd. for $C_{28}H_{27}N_3Cl_2O_6 \cdot CH_3SO_3H$:

C, 52.09; H, 4.67; N, 6.28; Cl, 10.61; S, 4.8.

Found: C, 51.87; H, 4.62; N, 6.14; Cl, 10.59; S, 4.81

## Example 17

(cis)-7-Chloro-1-[2-(dimethylamino)ethyl]-2,3,4,5-tetrahydro-4-(4-methoxyphenyl)-2-oxo-1H-1-benzazepin-3-yl methylcarbamate, monohydrochloride

A stirred suspension of 0.80 g (2.06 mmole) of (cis)-7-chloro-3-hydroxy-1-[2-(dimethylamino)-ethyl]-1,3,4,5-tetrahydro-4-(4-methoxyphenyl)-2H-1-benzazepin-2-one (see Example 1F) in 6 ml of dichloromethane was treated with 1.2 g (21 mmole) of methyl isocyanate and the resulting solution was allowed to stand at room temperature for 22 hours. TLC indicated the presence of a small quantity of starting material. The mixture was treated with 6 ml of acetonitrile and 1.2 g of methyl isocyanate and allowed to stand at room temperature for 20 hours. The solution was concentrated to give 1.1 g of a solid, which was dissolved in 35 ml of ethyl acetate, extracted with 5 ml of water (twice), dried over magnesium sulfate, filtered and the solvent evaporated to give 0.93 g of solid. This material was suspended in 5 ml of hexane and filtered to give 0.79 g of solid. This material was suspended in 5 ml of ether, cooled and

filtered to give 0.62 g of solid. This material was dissolved in 3 ml of ethanol and treated with 0.26 ml of 5.7N ethanolic hydrogen chloride and gradually diluted with about 50 ml of ether to give a crystalline product weighing 0.61 g, melting point 238-240°C, dec.

Analysis calc'd. for $C_{23}H_{28}N_3ClO_4 \cdot HCl \cdot H_2O$:

C, 56.21; H, 6.15; N, 8.55; Cl, 14.43

Found: C, 56.42; H, 6.14; N, 8.58; Cl, 14.48

Additional compounds falling within the scope of this invention include:

(cis)-3-(acetyloxy)-7-(difluoromethoxy)-1-[2-(dimethylamino)ethyl]-1,3,4,5-tetrahydro-4-(4-methoxyphenyl)-2H-1-benzazepin-2-one

(cis)-3-(acetyloxy)-7-(methylthio)-1-[2-(dimethylamino)ethyl]-1,3,4,5-tetrahydro-4-(4-methoxyphenyl)-2H-1-benzazepin-2-one

(cis)-3-(acetyloxy)-1-[2-(dimethylamino)ethyl]-1,3,4,5-tetrahydro-7-(methoxy)-4-(4-methoxyphenyl)-2H-1-benzazepin-2-one,monohydrochloride, melting point 215 - 217°C, dec., sintering at 205°C.

(cis)-3-(acetyloxy)-7-[(2,2-dimethylpropionyl)oxy]-1-[2-(dimethylamino)ethyl]-1,3,4,5-tetrahydro-4-(4-methoxyphenyl)-2H-1-benzazepin-2-one

(cis)-3-(acetyloxy)-7-[[(dimethylamino)-carbonyl]oxy]-1-[2-(dimethylamino)ethyl]-1,3,4,5-tetrahydro-4-(4-methoxyphenyl)-2H-1-benzazepin-2-one

(cis)-3-(acetyloxy)-7-chloro-1-[2-(dimethylamino)ethyl]-1,3,4,5-tetrahydro-4-[4-(methylthio)-phenyl]-2H-1-benzazepin-2-one

(cis)-1-[2-(Dimethylamino)ethyl]-1,3,4,5-tetrahydro-3-methoxy-4-(4-methoxyphenyl)-2H-1-benzazepin-2-one, monohydrochloride, melting point 223 - 224°C, dec.

(cis)-3-(Acetyloxy)-1-[2-(dimethylamino)-ethyl]-1,3,4,5-tetrahydro-7-[[(methylamino)-carbonyl]-oxy]-4-(4-methoxyphenyl)-2H-1-benzazepin-2-one, monohydrochloride, melting point 216 - 218°C, dec., sintering at 212°C.

(cis)-3-(Acetyloxy)-1-[2-(dimethylamino)-ethyl]-7-(2,2-dimethyl-1-oxopropoxy)-1,3,4,5-tetrahydro-4-(4-methoxyphenyl)-2H-1-benzazepin-2-one, monohydrochloride, melting point 198 - 200°C, dec., sintering at 160°C.

(cis)-3-(Acetyloxy)-1-[2-(dimethylamino)-ethyl]-2,3,4,5-tetrahydro-4-(4-methoxyphenyl)-2-oxo-1H-1-benzazepin-7-ol, dimethylcarbamate ester, monohydrochloride, melting point 178 - 181°C (bubbles), sintering at 168°C.

(cis)-3-(Acetyloxy)-1,3,4,5-tetrahydro-4-(4-methoxyphenyl)-1-[2-(1-pyrrolidinyl)ethyl]-7-(trifluoromethyl)-2H-1-benzazepin-2-one, monohydrochloride, melting point 228 - 230°C, dec., sintering at 225°C.

(cis)-3-(Acetyloxy)-7-chloro-1-[3-(dimethylamino)propyl]-1,3,4,5-tetrahydro-4-(4-methoxyphenyl)-2H-1-benzazepin-2-one, monohydrochloride, melting point 212 -215°C, dec, sintering at 200°C.

(d-cis)-3-(Acetyloxy)-7-chloro-1-[2-(dimethylamino)ethyl]-1,3,4,5-tetrahydro-4-(4-methoxyphenyl)-2H-1-benzazepin-2-one, monohydrochloride, melting point 252 -254°C, dec.

(l-cis)-3-(Acetyloxy)-7-chloro-1-[2-(dimethylamino)ethyl]-1,3,4,5-tetrahydro-4-(4-methoxyphenyl)-2H-1-benzazepin-2-one, monohydrochloride, melting point 253 - 255°C, dec.

(d-cis)-3-(Acetyloxy)-1-[2-(dimethylamino)ethyl]-1,3,4,5-tetrahydro-4-(4-methyoxyphenyl)-7-(trifluoromethyl)-2H-1-benzazepin-2-one, monohydrochloride, melting point 257 - 259°C, dec.

(l-cis)-3-(Acetyloxy)-1-[2-(dimethylamino)ethyl]-1,3,4,5-tetrahydro-4-(4-methoxyphenyl)-7-(trifluoromethyl)-2H-1-benzazepin-2-one, monohydrochloride, melting point 258 - 260°C, dec.

## CLAIMS

1. A compound having the formula

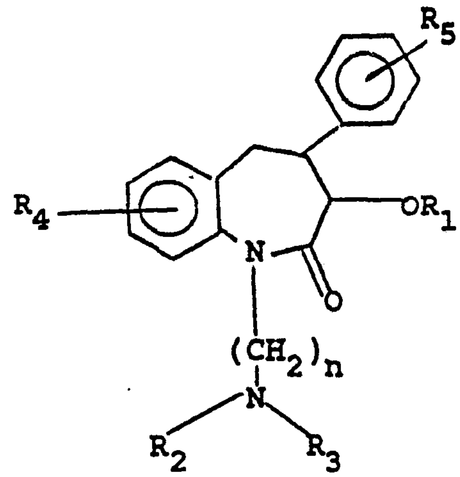

or a pharmaceutically acceptable salt thereof wherein

$R_1$ is hydrogen, alkyl, alkanoyl, alkenyl, arylcarbonyl, heteroarylcarbonyl or $-\overset{O}{\overset{\|}{C}}-NX_1X_2$;

$R_2$ and $R_3$ are each independently alkyl or cycloalkyl or $R_2$ and $R_3$ together with the nitrogen atom to which they are attached are pyrrolidinyl, piperidinyl, or morpholinyl;

$R_4$ and $R_5$ are each independently hydrogen, halogen, alkyl, alkoxy, aryloxy, arylalkoxy, arylalkyl, hydroxy, alkanoyloxy, $-O-\overset{O}{\overset{\|}{C}}-NX_1X_2$, difluoromethoxy, trifluoromethyl, $-NX_3X_4$, $-S(O)_m$alkyl, or $-S(O)_m$aryl;

n is 2 or 3;

m is 0, 1 or 2;

$X_1$ and $X_2$ are each independently hydrogen, alkyl, aryl or heteroaryl, or $X_1$ and $X_2$ together with the nitrogen atom to which they are attached are a nitrogen containing heteroaryl;

$X_3$ and $X_4$ are each independently alkyl, alkanoyl, arylcarbonyl, heteroarylcarbonyl, or carbamoyl;
with the proviso that if $R_4$ is a 7-alkyl group, it must have a tertiary carbon atom bonded to the ring.

2. A compound in accordance with claim 1 wherein $R_1$ is hydrogen.

3. A compound in accordance with claim 1 wherein $R_1$ is alkyl.

4. A compound in accordance with claim 1 wherein $R_1$ is alkanoyl.

5. A compound in accordance with claim 1 wherein $R_1$ is acetyl.

6. A compound in accordance with claim 1 wherein $R_1$ is alkenyl, arylcarbonyl. heteroarylcarbonyl or $-\overset{O}{\overset{\|}{C}}-NX_1X_2$.

7. A compound in accordance with any one of claims 1 to 6 wherein $R_2$ and $R_3$ are each alkyl.

8. A compound in accordance with any one or claims 1 to 6 wherein $R_2$ and $R_3$ are each methyl.

9. A compound in accordance with any one of claims 1 to 6 wherein $R_2$ and $R_3$ are each cycloalkyl.

10. A compound in accordance with any one of claims 1 to 6 wherein $R_2$ and $R_3$ together with the nitrogen atom to which they are attached are pyrrolidinyl, piperidinyl or morpholinyl.

11. A compound in accordance with any one of claims 1 to 10 wherein $R_4$ is hydrogen.

12. A compound in accordance with claim 1 wherein $R_4$ is halogen.

13. A compound in accordance with any one of claims 1 to 10 wherein $R_4$ is chlorine and is located in the 7-position of the benzazepine nucleus.

14. A compound in accordance with any one or claims 1 to 13 wherein $R_5$ is alkoxy.

15. A compound in accordance with any one of claims 1 to 13 wherein $R_5$ is methoxy and is located in the 4-position of the phenyl ring to which it is attached.

16. A compound in accordance with any one of claims 1 to 15 wherein n is 2.

17. A compound in accordance with any one of claims 1 to 15 wherein n is 3.

18. The compound in accordance with claim 1, (cis)-3-(acetyloxy)-7-chloro-1-[2-(dimethylamino)-ethyl]-1,3,4,5-tetrahydro-4-(4-methoxyphenyl)-2H-1-benzazepin-2-one, or a pharmaceutically acceptable salt thereof.

19. The compound in accordance with claim 1, (cis)-3-(acetyloxy)-1-[2-(dimethylamino)ethyl]-1,3,4,5-tetrahydro-4-(4-methoxyphenyl)-2H-1-benzazepin-2-one, or a pharmaceutically acceptable salt thereof.

20. The compound in accordance with claim 1, (trans)-3-(acetyloxy)-1-[2-(dimethylamino)ethyl]-1,3,4,5-tetrahydro-4-(4-methoxyphenyl)-2H-1-benzazepin-2-one, or a pharmaceutically acceptable salt thereof.

21. The compound in accordance with claim 1, (cis)-3-(acetyloxy)-1-[2-(dimethylamino)ethyl]-1, 3,4,5-tetrahydro-4-(4-methoxyphenyl)-7-(trifluoromethyl)-2H-1-benzazepin-2-one, or a pharmaceutically acceptable salt thereof.

22. The compound in accordance with claim 1, (cis)-3-(acetyloxy)-7-(methoxy)-1-[2-(dimethylamino)-ethyl]-1,3,4,5-tetrahydro-4-(4-methoxyphenyl) 2H-1-benzazepin-2-one, or a pharmaceutically acceptable salt thereof.